# EUROPEAN PATENT APPLICATION

(11) **EP 1 992 701 A2**
(43) Date of publication of application: **19.11.2008**
(21) Application number: 07023696.3
(22) Date of filing: 05.04.2002
(51) Int. Cl.: C12Q 1/68, C12P 19/34, C07H 21/04

(54) **Kits and methods for assessing oxidative stress**

(30) Priority: 05.04.2001 US 826522; 07.05.2001 US 289169; 22.10.2001 US 350517; 24.10.2001 US 335426; 05.12.2001 US 336815
(62) Divisional of application: 02736545.1
(71) Applicant: Genelink, Inc., Margate, NJ 08402 (US)
(72) Inventor: Dephillipo, John R., Margate NJ 08402 (US); Ricciardi, Robert P., Glenn Mills, PA 19342 (US)
(74) Representative: Grünecker, Kinkeldey, Stockmair & Schwanhäusser Anwaltssozietät

(57) **Abstract**

The invention relates to kits and methods for assessing the susceptibility of a human to oxidative stress or damage. The methods involve assessing occurrence in the human's genome of one or more polymorphisms (e.g., single nucleotide polymorphisms) that occur in one or more genes associated with oxidative stress and that are associated with a disorder in humans. Preferred assessment and scoring methods are disclosed, as are kits for performing the methods.

## Description

### BACKGROUND OF THE INVENTION

Oxidation of the chemical components of foodstuffs provides energy that is used to build and maintain the body and to enable normal physiological function. Such oxidation involves a chain of chemical reactions including reactions in which transfer of electrons from one chemical compound to another are catalyzed. These reactions are catalyzed by enzymes, which serve to align and chemically activate one or more of, for example, reactants, cofactors, metal atoms or ions, and water molecules. Despite the inherent specificity of enzyme-catalyzed reactions, side reactions inevitably occur.

Oxygen is a common and relatively chemically reactive component of biological systems. Diatomic oxygen is ordinarily relatively harmless to body systems, as is fully reduced oxygen (i.e., water). However, transfer of one or more electrons to oxygen (e.g., during reduction of oxygen to water during oxidative phosphorylation or by way of a side reaction of another biochemical process) can result in formation of more reactive species of oxygen, such as hydrogen peroxide, superoxide radicals, and hydroxyl radicals. These relatively reactive forms of oxygen can damage biochemical components of the body such as proteins, lipids, and DNA, destroying or inhibiting the normal function of the components.

The effects of biochemical damage inflicted by interaction of reactive forms of oxygen with body components can be manifested in a number of ways. DNA is the genetic material that carries the 'instructions' for making the components of a normal human body. Oxidative damage to DNA can result in mutations (i.e., changes in the 'instructions') that lead the body to make abnormal components. The abnormal components can have inhibited (or no) ability to perform their normal function, and this can be manifested as a disease or disorder. Likewise, oxidative damage to enzymes or lipid components of membranes can inhibit or ablate their normal function, and this too can be manifested as a disease or disorder. The degree to which a cell or tissue of a human body is subjected to damage caused by reactive forms of oxygen is sometimes designated 'oxidative stress.' The diseases and disorders associated with oxidative damage to body components are thus manifestations of oxidative stress. Aging is another manifestation of oxidative stress. Over time, damage caused by interaction of reactive forms of oxygen with body components degrades the structure and function of those components, leading to detectable changes in body structure and function.

If the human body were not able to detoxify reactive forms of oxygen and mitigate their effects on the body, then human life would be significantly shorter or even impossible. However, the human body comprises enzymes which are able to catalyze transformation of reactive forms of oxygen to less toxic species and other enzymes which are able to repair damage done to body components by reactive forms of oxygen.

Human immune reactions in response to pathogenic infection include activation of macrophages and polymorphonuclear neutrophilic granuloctyes. Activation of these and other immune cells enhances production of reactive oxygen species (e.g., superoxide radicals and hydrogen peroxide) by the cells. These reactive oxygen species exert an anti-infective effect by damaging the infecting cells, but can also harm host tissues, particularly if their production is not closely regulated. Some organisms, such as Bacillus anthracis, are able to induce overproduction of reactive oxygen species to the extent that systemic shock, or even death, is induced in the host. The toxic effects of reactive oxygen species generated by immune cells in response to infection can be limited or prevented by production in the host of anti-oxidant compounds (e.g., glutathione) or enzymes that catalyze detoxification of reactive oxygen species or by administration of an anti-oxidant compound or precursor (e.g., N-acetyl-cysteine) to an infected (or potentially infected) host.

Defects in the ability of immune system cells to produce appropriate amounts of reactive oxygen species are know to be associated with human disorders such as chronic granulomatous disease (CGD). Patients afflicted with CGD exhibit a marked reduction in the ability of their macrophages to produce microbicidally effective amounts of reactive oxygen species (or a complete absence of that ability), and are unusually susceptible to pathogenic infections.

Most, if not all, human genes occur in a variety of forms which differ in at least minor ways. Heterogeneity in human genes is believed to have arisen, in part, from minor, non-fatal mutations that have occurred in the genome over time. In some instances, differences between alternative forms of a gene are manifested as differences in the amino acid sequence of a protein encoded by the gene. Some amino acid sequence differences can alter the reactivity or substrate specificity of the protein. Differences between alternative forms of a gene can also affect the degree to which (if at all) the gene is expressed. However, many heterogeneities that occur in human genes appear not to be correlated with any particular phenotype. Known heterogeneities include, for example, single nucleotide polymorphisms (i.e., alternative forms of a gene having a difference at a single nucleotide residue). Other known polymorphic forms include those in which the sequence of larger (e.g., 2-1000 residues) portions of a gene exhibits numerous sequence differences and those which differ by the presence or absence of portion of a gene.

Numerous disorders and physiological states have been correlated with occurrence of one or more alternative forms of a gene in the genome of a human who exhibits the disorder or physiological state. For example, Kimura et al. (2000, Am. J. Ophthalmol. 130:769-773) discloses an association between occurrence of a SNP of the manganese superoxide dismutase gene and a form of macular degeneration. Although associations between individual disorders and individual genetic polymorphisms are known, a need remains for a method of assessing the overall state of oxidative stress to which a human is subjected. The invention satisfies this need.

Topically applied skin care products (e.g., cremes such as facial and hand cremes, lotions such as sun tan lotions, antiseptic compositions, anti-itch compositions, shaving cremes and gels, shampoos and soaps, astringents, and the like) sometimes contain antioxidant ingredients such as vitamin E. However, there does not appear to be any systematic method of determining whether any particular individual should employ a skin care product containing an antioxidant ingredient. For this reason, significant damage can be done to the skin of individuals who are especially susceptible to oxidative damage, and such damage often is not detected until the outward appearance of the skin changes. By this point, much of the damage already inflicted cannot be repaired. A need exists for a method of identifying whether it is advantageous for an individual to employ a skin care product comprising an antioxidant ingredient prior to infliction of oxidative damage. The invention satisfies this need as well.

### BRIEF SUMMARY OF THE INVENTION

The invention relates to a method of assessing relative susceptibility of a human to oxidative damage. The method comprises assessing occurrence in the human's genome of disorder-associated polymorphisms (e.g., single nucleotide polymorphisms; SNPs) in at least two (and preferably three, four, six, ten, fifteen, or twenty or more) genes selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress;
f) genes which encode a component of the human DNA repair system; and
g) genes which encode a protein associated with production of a toxic oxygen species by a macrophage or polymorphonuclear neutrophilic granulocyte and which are not a gene of groups a), b), c), d), e), or f).
Substantially the same method can be used to assess the advisability that a human should employ a skin care product comprising an antioxidant ingredient.

Occurrence of any of the polymorphisms is an indication that the human is more susceptible to oxidative damage than a human whose genome does not comprise the polymorphism. When the method is used to assess the advisability that a human should employ a skin care product comprising an antioxidant ingredient, occurrence of any of the polymorphisms is an indication that it is more advisable for the human to use the product containing the antioxidant than when the polymorphism(s) do not occur, or that a skin care product containing a greater amount of the antioxidant ingredient should be used than when the polymorphism does not occur. Furthermore, occurrence of a plurality of the polymorphisms is an indication that the human is even more susceptible to oxidative damage than a human whose genome does not comprise the polymorphisms (or that it is even more advisable that a skin care product containing an antioxidant ingredient or that a skin care product containing a greater amount of the antioxidant ingredient should be used than when the plurality of polymorphisms does not occur). Preferably the genes are selected from the group consisting of a), b), c), and d), and more preferably they are selected from the group consisting of a), b), and c). In one embodiment, the method comprises assessing occurrence in the human's genome of disorder-associated polymorphisms in at least four genes selected from the group consisting of genes which encode an enzyme that catalyzes conversion of a toxic oxygen species to a less toxic oxygen species (e.g., genes which encode mitochondrial manganese superoxide dismutase, cytoplasmic copper/zinc superoxide dismutase, catalase, and glutathione peroxidase).

In another embodiment, the genes include at least one gene associated with production of a toxic oxygen species by a macrophage or polymorphonuclear neutrophilic granulocyte. In this embodiment, the assay can be used to assess the susceptibility of the human to the type of oxidative stress that is associated with reaction of the human's immune system to a microbial infection. For example, the susceptibility of the human to oxidative stress associated with an immune reaction prompted by a bacterial infection (e.g., infection by Bacillus anthracis, Staphylococcus aureus, or Escherichia coli).

In still another embodiment, occurrence of a polymorphism in at least one gene from group e) is assessed. For example, occurrence of a polymorphism can be assessed in a gene that encodes a matrix metalloproteinase (MMP), such as the gene that encodes MMP-1, MMP-2, MMP-3, or MMP-7 (e.g., the 1G/2G polymorphism that occurs at base pair 1607 of the gene that encodes MMP-1).

In another embodiment, occurrence of a polymorphism in at least one gene from group d) is assessed. For example, occurrence of a polymorphism can be assessed in a gene that encodes a glutathione S-transferase, such as the gene that encodes glutathione S-transferase P1, glutathione S-transferase theta 1, or glutathione S-transferase M1 (e.g., the polymorphism that occurs at one of amino acid residues 21, 141, and 169 of glutathione S-transferase theta 1). As another example, occurrence of a polymorphism can be assessed in the gene that encodes tumor necrosis factor alpha (e.g., the polymorphism that occurs at one of nucleotide residues -238 and -308 of the TNFA gene) or in the gene that encodes methylenetetrahydrofolate reductase.

The method by which occurrence of an individual disorder-associated polymorphism is assessed is not critical. For example, occurrence of the polymorphisms can be assessed using a method that includes contacting a nucleic acid derived from the human's genome with a first oligonucleotide. The first oligonucleotide can be one that anneals with higher stringency with the disorder-associated polymorphism than with a corresponding non-disorder-associated polymorphism. Annealing of the first oligonucleotide and the nucleic acid can be assessed, and such annealing is an indication that the human's genome comprises the disorder-associated polymorphism. Use of an oligonucleotide has the advantage that the oligonucleotide can be attached to a support using routine methods, and that a plurality of oligonucleotides can be attached to the same support, to allow simultaneous detection of multiple polymorphisms. If a second oligonucleotide which anneals with higher stringency with a non-disorder-associated polymorphism than with a corresponding disorder-associated polymorphism is used, then the allelic content of the human's genome can be determined. Detection of polymorphic sequences can be simplified by using labeled oligonucleotides, such as molecular beacon oligonucleotides.

Once the content of the human's genome for disorder-associated polymorphisms has been assessed, assessment of susceptibility to oxidative damage (or advisability of using a skin care product comprising an antioxidant ingredient) can further comprise calculating a susceptibility score for the human. A susceptibility score can be calculated by summing, for each of the selected genes in which a disorder-associated polymorphism occurs in the human's genome, the product of a constant and a correlation factor. The correlation factor can, alternatively, be a factor that represents the fraction of humans heterozygous for the disorder-associated polymorphism who exhibit the corresponding disorder or a factor that represents the fraction of humans homozygous for the disorder-associated polymorphism who exhibit the corresponding disorder. The constant can be selected based on the known or surmised relevance of the gene with respect to oxidative damage. The susceptibility score represents the relative susceptibility of the human to oxidative damage. Likewise, a greater susceptibility score corresponds to a greater advisability that the human should employ a skin care product comprising an antioxidant ingredient than does a lower score, and a greater score can also indicate that the human should use a skin care product the contains a greater amount of such an ingredient.

In another aspect, the invention relates to a method of selecting a dose of an anti-oxidant composition (i.e., a composition comprising a compound that exhibits anti-oxidant properties, such as vitamin E or vitamin C, or a compound that can otherwise supplement the body's normal anti-oxidant mechanisms, such as alpha-lipoic acid and coenzyme Q) for administration to a human. This method comprises assessing occurrence in the human's genome of disorder-associated polymorphisms in at least one of the genes selected from the group consisting of a), b), c), d), e), and f), as indicated above. After assessing occurrence of the polymorphisms, a dose of the composition is selected. Occurrence of any of the polymorphisms is an indication that a greater dose of the composition should be administered to the human.

The invention also relates to a kit for assessing relative susceptibility of a human to oxidative damage. The kit comprises reagents for assessing occurrence in the human's genome of disorder-associated polymorphisms in at least one gene selected from the group consisting of a), b), c), d), e), and f), as indicated above. Examples of suitable reagents include oligonucleotides (e.g., molecular beacon oligonucleotides) that anneal with higher stringency with the disorder-associated polymorphisms than with corresponding non-disorder-associated polymorphisms and oligonucleotide primers that are complementary to the region adjacent a characteristic residue of the disorder-associated polymorphism. These primers are useful for amplifying at least the characteristic residue, thereby facilitating its detection. The kit can further comprise an instructional material which includes a numerical value representing the product of a constant and a correlation factor.

In another aspect, the invention relates to a method of selecting a dose of an anti-oxidant composition in a skin care product (i.e., a skin care product comprising a compound that exhibits anti-oxidant properties, such as vitamin E or vitamin C, or a compound that can otherwise supplement the body's normal anti-oxidant mechanisms, such as alpha-lipoic acid and coenzyme Q) for administration to a human. This method comprises assessing occurrence in the human's genome of disorder-associated polymorphisms in at least one of the genes selected from the group consisting of a), b), c), d), e), and f), as indicated above. After assessing occurrence of the polymorphisms, a dose of the composition is selected.
Occurrence of any of the polymorphisms is an indication that a greater dose of the composition should be administered to the human in the skin care product

### BRIEF DESCRIPTION OF THE SEVERAL VIEWS OF THE DRAWINGS

The foregoing summary, as well as the following detailed description of preferred embodiments of the invention, will be better understood when read in conjunction with the appended drawings. The invention is not limited to the precise arrangements and instrumentalities shown.

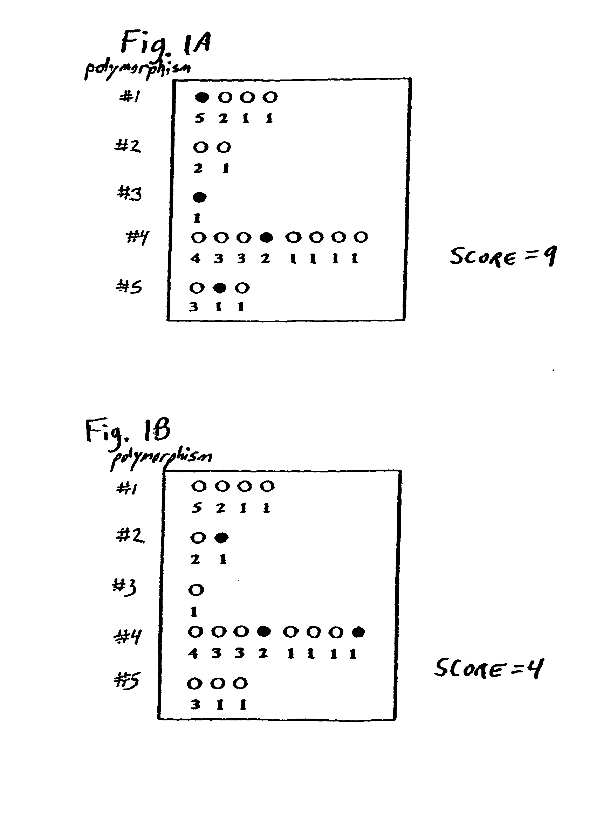

Figures 1A and 1B are images which depict examples of results that can be obtained by analyzing occurrence of polymorphisms in several genes. The results shown in Figure 1A are derived from a hypothetical first human, and those shown in Figure 1B are derived from a hypothetical second human. Circles represent different polymorphisms of the gene indicated to the left of the row of circles. Filled circles indicate the presence of the polymorphism. Non-filled circles indicate the absence of the polymorphism. Numbers below each circle represent a correlation factor for the polymorphism and a disease or disorder.

### DETAILED DESCRIPTION OF THE INVENTION

The invention relates to kits and methods for assessing the relative susceptibility of a human to oxidative damage by assessing occurrence in the human's genome of genetic polymorphisms that are associated with disorders.

Crudely simplified, the methods involves determining whether one or more polymorphisms that have been associated (by the inventors or by others) with a disorder (e.g., a disease or pathological state) in humans occur in the genome of the human being tested. In some embodiments, the number of polymorphisms that occur in the human's genome are summed to yield a value; the higher the value is, the greater the susceptibility of the human to oxidative damage is assessed to be. In other embodiments, a weighting factor is assigned to each polymorphism tested, and the weighting factors of polymorphisms that occur in the human's genome are summed to yield a value that represents relative susceptibility to oxidative damage. The weighting factor can represent the product of a constant assigned to the gene in which the corresponding polymorphism occurs and a correlation factor that describes how informative occurrence of the polymorphism is for occurrence of the disorder with which it is associated. The invention includes a variety of alternative methods and kits for performing the methods, as described in greater detail herein.

Definitions

As used in this disclosure, the following terms have the meanings associated with them in this section.

A "polymorphism" in a gene is one of the alternative forms of a portion of the gene that are known to occur in the human population. For example, many genes are known to exhibit single nucleotide polymorphic forms, whereby the identity of a single nucleotide residue of the gene differs among the forms. Each of the polymorphic forms represents a single polymorphism, as the term is used herein. Other known polymorphic forms include alternative forms in which multiple consecutive or closely-spaced, non-consecutive nucleotide residues vary in sequence, forms which differ by the presence or absence of a single nucleotide residue or a small number of nucleotide residues, and forms which exhibit different mRNA splicing patterns.

A "single nucleotide polymorphism" ("SNP") is one of the alternative forms of a portion of a gene that vary only in the identity of a single nucleotide residue in that portion.

A "disorder-associated" polymorphism is an alternative form of a portion of a gene, wherein occurrence of the alternative form in the genome of a human has been correlated with exhibition by the human of a disease or a pathological state.

A "non-disorder-associated" polymorphism is an alternative form of a portion of a gene for which no significant correlation has been made between occurrence of the alternative form in the genome and a disease or a pathological state. Non-disorder-associated polymorphisms are sometimes designated "neutral" polymorphisms in the art.

A disorder-associated polymorphism and a non-disorder-associated polymorphism "correspond" with one another if the two polymorphisms are two alternative forms of the same portion of the gene. By way of example, if the identity of residue 100 of a gene is adenine in a disorder-associated polymorphism of the gene and cytosine in a non-disorder-associated polymorphism of the gene, then the two polymorphisms correspond with one another. It is understood that there may be three or more corresponding polymorphisms when there are more than two alternative forms of the same portion of the gene.

A "characteristic residue" of a polymorphism is a nucleotide residue, the identity of which is known to vary among the alternative forms corresponding to the polymorphism.

"Toxic oxygen species" include, in approximate order of reactivity, hydroxyl radicals, superoxide radicals, nitric oxide, peroxy nitrite (ONOO-; the product of a reaction between nitric oxide and superoxide radical), and hydrogen peroxide. Ordinary diatomic oxygen is not a toxic oxygen species, as the term is used herein.

"Oxidative damage" refers to chemical reaction of a normal cellular component (e.g., DNA, a protein, or a lipid) with a toxic oxygen species, whereby at least one normal function of the component is inhibited or eliminated. The terms "oxidative damage" and "oxidative stress" are used interchangeably herein.

A "molecular beacon oligonucleotide" is a single-stranded oligonucleotides having a fluorescent label (e.g., rhodamine, FAM, TET, VIC, JOE, or HEX) attached to the 5'-end thereof and a fluorescence quencher (e.g., TAMRA or DABCYL) attached to the 3'-end thereof (or vice versa), as described (Kostrikis et al., 1998, Science 279:1228-1229).

Two molecular beacon oligonucleotides are "spectrally distinct" if they can be differentially detected using spectrophotometric or spectrofluorimetric methods. Examples of characteristics that can be used to differentiate spectrally distinct oligonucleotides include absorption or excitation wavelength, emission wavelength, and fluorescent lifetime.

An "instructional material" is a publication, a recording, a diagram, or any other medium of expression which can be used to communicate how to use a kit described herein, numerical values for weighting the significance of various polymorphisms that are detectable using the kit, or both. The instructional material of the kit of the invention can, for example, be affixed to a container which contains a kit of the invention or be shipped together with a container which contains the kit. Alternatively, the instructional material can be shipped separately from the container with the intention that the instructional material and the kit be used cooperatively by the recipient.

The "stringency" with which two polynucleotides anneal means the relative likelihood that the polynucleotides will anneal in a solution as the conditions of the solution become less favorable for annealing. Examples of stringent conditions are known in the art and can be found in available references (e.g., Current Protocols in Molecular Biology, John Wiley & Sons, N.Y., 1989, 6.3.1-6.3.6). Aqueous and non-aqueous annealing methods are described in that reference and either can be used. In general, a first pair of polynucleotides anneal with higher stringency than a second pair if the first pair is more likely to anneal (or remain annealed) as one or more of the salt concentration, temperature, and detergent concentration are increased.

With respect to a disorder, a "correlation factor" for a disorder-associated polymorphism is the fractions of humans who are heterozygous or homozygous for the polymorphism who exhibit the disorder. The correlation factor can, alternatively, be based solely on those who are heterozygous, solely on those who are homozygous, or on those who are either heterozygous or homozygous.

A "non-extendable" nucleotide residue is a nucleotide residue that is capable of being added to a polynucleotide by a polymerase (i.e., by extension of the polynucleotide in association with a complement thereof, catalyzed by the polymerase) and that, upon addition to the polynucleotide, renders the polynucleotide incapable of being further extended by the polymerase.

Description

The invention relates to kits and methods for assessing the relative susceptibility of a human to oxidative damage by assessing occurrence in the human's genome of genetic polymorphisms that are associated with disorders.

It has been discovered that the degree to which a human is susceptible to oxidative damage can be assessed by determining which polymorphic forms of certain genes are present in the human's genome. The genes which are assessed are genes that are associated with oxidative stress, including both genes which provide protection against oxidative damage and genes which exacerbate oxidative damage.

Among the types of genes which protect the body against oxidative stress are genes which encode an enzyme that catalyzes conversion of a toxic oxygen species to a less toxic oxygen species, genes that encode a protein that directly provides protection against oxidative damage, genes which encode a protein that indirectly provides protection against oxidative damage, genes which encode a component of the human DNA repair system, and genes (not necessarily included within the preceding groups) which are associated with inducible production of reactive oxygen species in immune cells upon microbial infection.

Numerous genes encode components of the human DNA repair system, and disorder-associated polymorphisms in substantially any of these genes can be informative of the susceptibility of the individual to oxidative stress. Examples of these genes include those which encode apurinic and apyrimidinic endonucleases, enzymes that catalyze excision of nucleotide residues damaged by ultraviolet radiation, and enzymes that catalyze site specific-recombination. Many such genes are known, and include those listed in Wood et al., 2001, Science 291(5507):1284-1289.

Genes that induce production of reactive oxygen species in immune cells upon microbial infection include genes (e.g., genes which encode components of the human phagocyte-specific NADPH-oxidase complex) associated with respiratory burst (sometimes designated oxidative burst) phenomena of macrophages and polymorphonuclear nucleophilic granulocytes, whereby toxic oxygen species are produced in response to invasion of a tissue by a microbe (e.g., a protozoan, or a bacterium such as a Pseudomonas, Salmonella, or Serratia bacterium or a known pathogen such as Bacillus anthracis, Escherichia coli, or Staphylococcus aureus). Also included within this group are genes which are known to be aberrant in patients afflicted with disorders that inhibit or abolish antimicrobial activities of macrophages (e.g., chronic granulomatous disease).

It is known (e.g., Hanna et al., 1994, Mol. Med. 1:7-18) that toxic oxygen species produced by components of the immune system in response to infection by certain organisms, such as Bacillus anthracis, can have undesirable effects on the human body. In patients afflicted with B. anthracis, these toxic species can induce systemic shock, or even death. Timely administration of anti-oxidant compounds can mitigate these effects, and administration of anti-oxidants prior to exposure can inhibit or even prevent them, particularly in individuals who exhibit a high susceptibility to oxidative stress.
Identification of such individuals (e.g., using the methods described herein) can be used to assess whether individuals at risk of exposure to B. anthracis or other pathogens should be administered an anti-oxidant prior to an anticipated or possible exposure.

Genes that encode enzymes that catalyze reactions responsible for decreasing electrophilic potential of allergens (or their metabolites), a process designated biotransformation of allergens, also affect the production of reactive oxygen species produced in immune cells (e.g., by modulating the rate at which allergens are removed from the body). Members of the glutathione S-transferase (GST) family of enzymes, such as GST theta 1 (GSTT1), GSTM1, and GSTP1, participate in the biotransformation of allergens. These enzymes also catalyze interconversions among more and less reactive forms of oxygen. Occurrence of one or more polymorphism in one of these GST genes can therefore be used to assess the susceptibility of a human to oxidative stress. For example, Lutz et al. indicate the presence of GSTT1 and GSTM1 enzymes in skin and state that carriers of defective one or both of the GSTT1 and GSTM1 genes is associated with increased vulnerability to allergenic effects of allergens (Lutz et al., 2001, Med. Pr. 52:45-51).

Another enzyme involved in production of toxic oxygen species by components of the immune system in response to allergen exposure is tumor necrosis factor alpha (TNFA). Allen et al. (2000, Immunogenetics 51:201-205) described a polymorphism that occurs at base pair -308 (i.e., in the promoter region) of the gene that encodes TNFA. This polymorphism affects an individual's response to a skin irritant. Assessing which of the two forms of this disorder-associated polymorphism occurs in alleles of a human's TNFA gene allows assessment of the human's susceptibility to oxidative stress (e.g., that resulting from the immune response induced by skin irritant exposure).

Among enzymes that catalyze conversion of a toxic oxygen species to a less toxic oxygen species, four are of particular relevance, namely mitochondrial manganese superoxide dismutase (MnSOD), cytoplasmic copper/zinc superoxide dismutase (CZSOD), catalase (CAT), and glutathione peroxidase (GP). Polymorphisms that occur in these genes are known to be associated with various disorders (see, e.g., Kimura et al., 2000, Am. J. Ophthalmol. 130:769-773). Occurrence of disorder-associated polymorphisms in at least one (and preferably two, three, or all) of these four genes should be assessed in the methods described herein, given the importance of these genes. Similarly, the kits described herein preferably include reagents for detecting di disorder-associated polymorphisms in at least one (and preferably two, three, or all) of these four genes. In addition, the significance of occurrence of disorder-associated polymorphisms in these genes can be applied by assigning a greater weighting factor to disorder-associated polymorphisms of these genes than to disorder-associated polymorphisms in other genes associated with oxidative stress.

It was not previously appreciated that detection in a human's genome of two or more disorder-associated polymorphisms in genes associated with oxidative stress is indicative that the human globally exhibits enhanced susceptibility to oxidative damage. Previous studies are believed to have recognized only association between a polymorphism in one of these genes and a particular disorder (e.g., exudative macular degeneration in the Kimura reference). The inventors believe that they are the first to describe methods and kits for assessing a human's global (i.e., not limited to a particular tissue, cell type, or organ) susceptibility to oxidative damage.

In addition to the MnSOD, CZSOD, CAT, and GP genes mentioned above, other genes encode proteins which provide direct or indirect protection against oxidative damage, for example by converting toxic species of oxygen to less toxic species, by eliminating precursors of toxic forms of oxygen, or by repairing oxidative damage. Examples of these genes include those which encode glutathione S-transferase P1, glutathione S-transferase theta 1, glutathione S-transferase M1, glutathione reductase, thioredoxin reductase, paraoxonase, NAD(P)H:quinone oxidoreductases 1 and 2, 8-oxo-7,8-dihydrodeoxyguanosine triphosphatase, and epoxide hydrolase. Detection in a human genome of disorder-associated polymorphisms in one or more of these genes indicates that the human exhibits enhanced susceptibility to oxidative damage. The methods and kits described herein can use this indication to assess the susceptibility of a human to oxidative stress.

Among the genes which exacerbate oxidative damage are genes which encode a protein that induces production of a toxic oxygen species, either directly (e.g., by catalyzing a reaction in which a toxic species of oxygen is a direct or side product) or indirectly (e.g., by enhancing flux through a metabolic pathway that leads to production of a toxic species of oxygen). Examples of proteins that directly or indirectly induce production of toxic oxygen species include myeloperoxidase, tumor necrosis factor alpha, NADH/NADPH oxidase p22 phox protein, nitric oxide synthase xanthine oxidase, and cytochrome P450. Detection in a human genome of disorder-associated polymorphisms in one or more genes encoding one of these proteins indicates that the human exhibits enhanced susceptibility to oxidative damage.

The methods described herein can also be used to assess susceptibility to oxidative damage by determining the presence in a human's genome of polymorphic forms of genes that are associated with oxidative damage, regardless of whether the mechanism by which the gene affects oxidative stress is understood. By way of example, apolipoprotein E is a multi-functional molecule that is able to affect oxidative stress. The ApoE4 phenotype, for example, is known to be associated with enhanced hydroxyl radical levels in patients afflicted with Alzheimer's disease, and ApoE expression is known to exacerbate oxidative stress. Further by way of example, enhancement of oxidative stress is known to be associated with each of elevated homocysteine level, depressed serum bilirubin level, depressed acid phosphatase activity, depressed protein phosphotyrosine phosphatase activity, and depressed epinephrine oxidase activity. Thus, occurrence in the genome of polymorphisms in genes which encode proteins that affect these levels and activities can be determined, and their occurrence can be used to estimate susceptibility of the human to oxidative stress. Examples of genes for which polymorphisms can be associated with altered susceptibility to oxidative damage include UDP-glucuronosyltransferase 1A1 (i.e., the UGT1A1 gene), genes encoding acid phosphatase, protein phosphotyrosine phosphatase, epinephrine oxidase, ApoE4, cystathionine beta-synthase, cystathionine gamma-lyase, N₅-methyl THF:homocysteine methyltransferase, methylenetetrahydrofolate reductase (MTHFR), and S-adenosylmethionine methyltransferase. Ueland et al. (2001, Trends Pharmacol. Sci. 22:195-201) described a polymorphism which occurs at base pair 677 of the gene that encodes MTHFR. This polymorphism affects folate distribution, thereby affecting DNA synthesis and homocysteine levels. Assessing which of the forms of this disorder-associated polymorphism occurs in the alleles of a human's MTHFR gene allows assessment of the human's susceptibility to damage attributable to oxidative stress.

Heat shock proteins are also known to provide at least indirect protection of cells from oxidative damage, and occurrence of a heat shock protein gene polymorphism can be used as informative markers of susceptibility to oxidative damage when the polymorphism is known to be a disorder-associated polymorphism.

It is not critical that the gene in which the occurrence of a polymorphism occurs is directly or indirectly involved in production or destruction of reactive oxygen species in the body. It is sufficient if an association can be made between the level of expression of a gene and the level of oxidative stress in the human. By way of example, Aparna et al. (2001, J. Biol. Chem. 276(17):14264) have described an association between the level of expression of matrix metalloproteinase (MMP) genes and oxidative stress in humans. Expression of genes encoding MMPs designated MMP-1, MMP-2, MMP-3, and MMP-7 is enhanced in the presence of a greater steady-state level of hydrogen peroxide than in the presence of a lower level. This observation indicates that expression of at least these four MMP genes is associated with the level of oxidative stress in a human. Occurrence of a polymorphism in one of these MMP genes can therefore be used to assess the susceptibility of a human to oxidative stress. For example, Aparna et al. described a polymorphism (1 G/2G) that occurs at base pair 1607 of the gene that encodes MMP-1. This polymorphism affects the level of expression of the MMP-1 gene in response to hydrogen peroxide level. Thus, assessing which of the two forms of this polymorphism occurs in the alleles of a human's MMP-1 gene allows assessment of the human's susceptibility to oxidative stress.

Examples of the polymorphisms in the foregoing genes which can be informative for susceptibility to oxidative damage include the following:
■ a polymorphism manifested as a change from an alanine residue to a valine residue at amino acid residue 9 (i.e., in the signal sequence) of MnSOD;
■ a polymorphism manifested as a change from an isoleucine residue to a thymine residue at amino acid residue 58 of MnSOD;
■ a polymorphism manifested as a change from a valine residue to a glutamic acid residue at amino acid residue 7 of CZSOD;
■ a polymorphism manifested as a change from a cysteine residue to a phenylalanine residue at amino acid residue 6 of CZSOD;
■ a polymorphism manifested as a change from a cytosine residue to a thymine residue at nucleotide residue -262 (i.e., in the promoter region) of the catalase gene;
■ a polymorphism in the hGPX1 gene manifested as a change from a proline residue to a leucine residue at amino acid residue 198 of glutathione peroxidase;
■ a polymorphism in the GSTP1 gene manifested as a change from a valine residue to an isoleucine residue at amino acid residue 105 of glutathione S-transferase P1;
■ a polymorphism in the GSTT1 gene manifested as a change from an alanine residue to a threonine residue at amino acid residue 21 of glutathione S-transferase theta 1;
■ a polymorphism in the GSTT1 gene manifested as a change from an aspartic acid residue to a asparagine residue at amino acid residue 141 of glutathione S-transferase theta 1;
■ a polymorphism in the GSTT1 gene manifested as a change from a valine residue to a isoleucine residue at amino acid residue 169 of glutathione S-transferase theta 1;
■ a polymorphism in the GSTM1 gene manifested as a change from a lysine residue to a asparagine residue at amino acid residue 173 of glutathione S-transferase M1;
■ a polymorphism manifested as a change from cytosine residue to a thymine residue at nucleotide residue 677 of the gene which encodes methylenetetrahydrofolate reductase (MTHFR);
■ a polymorphism manifested as a change from a thymine residue to a cytosine residue at nucleotide residue -107 (i.e., in the promoter region) of the gene which encodes paraoxonase;
■ a polymorphism manifested as a change from a cytosine residue to a thymine residue at nucleotide residue 242 (i.e., in the coding region) of the gene encoding NAD(P)H:quinone oxidoreductase;
■ a polymorphism manifested as a change from a thymine residue to a cytosine residue at nucleotide residue 113 in exon 3 of the gene which encodes epoxide hydrolase (i.e., effecting change of from a tyrosine residue to a histidine residue in epoxide hydrolase);
■ a polymorphism manifested as a change from a guanine residue to an adenine residue at nucleotide residue -463 (i.e., in the promoter region) of the gene which encodes myeloperoxidase;
■ a polymorphism manifested as a change to an adenine residue at nucleotide residue -238 (i.e., in the promoter region) of the gene which encodes tumor necrosis factor alpha (i.e., the TNF promoter variant designated TNF2);
■ a polymorphism manifested as a change to an adenine residue at nucleotide residue -308 (i.e., in the promoter region) of the gene which encodes tumor necrosis factor alpha (i.e., the TNF promoter variant designated TNF3);
■ a polymorphism manifested as a change from a cytosine residue to a thymine residue at nucleotide residue 242 (i.e., in the coding region) of the phox gene encoding the NADH/NADPH oxidase p22 subunit;
■ a polymorphism manifested as a 27 base pair repeat in intron 4 (i.e., between nucleotide residues 5130 and 5511) of the gene encoding nitric oxide synthase;
■ a polymorphism manifested as a change from an adenine residue to a guanine residue at nucleotide residue -290 (i.e., in the 5'-flanking region) of the gene encoding cytochrome P450 (i.e., the polymorphism designated the CYP3A4 cytochrome P450 variant);
■ the polymorphism designated the ApoE4 allele of the ApoE gene;
■ a polymorphism manifested as a change from a cytosine residue to a thymine residue at nucleotide residue 699 (i.e., in the coding region) of the gene encoding cystathionine beta-synthase
■ the 1G/2G polymorphism that occurs at base pair 1607 (using the numbering of Aparna et al.) of the human gene encoding MMP-1.

Methods of Assessing Susceptibility to Oxidative Damage

The invention includes a method of assessing the relative susceptibility of a human to oxidative damage. This susceptibility can be calculated relative to a hypothetical human whose genome does not contain a single disorder-associated polymorphism in a gene associated with oxidative stress. Alternatively, susceptibility can be calculated relative to another human who may have one or more different disorder-associated polymorphism than the human being assessed. In practice, the basis upon which raw susceptibility scores are calculated is immaterial, so long as the same basis is used for all humans whose scores are to be compared (i.e., so that the scores are relatable to one another).

The relative susceptibility of a human to oxidative damage permits assessment of risks and benefits of a variety of compositions, conditions, and interventions. In one embodiment, the susceptibility of a human to oxidative damage can be used to determine whether the human would benefit by supplementing nutritional intake with a composition that contains one or more anti-oxidants. Furthermore, the relative susceptibility of the human to oxidative damage can indicate an appropriate dose of such an anti-oxidant-containing composition. In another embodiment, suitability of a condition or intervention for a human (e.g., administration to the human of hyperbaric oxygen or a pharmaceutical agent known to induce generation of toxic species of oxygen) can be determined by assessing the human's susceptibility to oxidative damage.

Susceptibility of a human to oxidative damage is assessed by assessing occurrence in the human's genome of disorder-associated polymorphisms in a plurality of genes associated with oxidative stress (e.g., 3, 4, 6, 8, 10, 15, 20, or 30 or more genes). Occurrence of a disorder-associated polymorphism in one of these genes is an indication that the human has a greater susceptibility to oxidative damage than a human in whose genome the polymorphism does not occur. Of course, occurrence of two or more such polymorphisms in the human's genome indicates that the human exhibits even greater susceptibility to oxidative damages.

Occurrence of every disorder-associated polymorphisms in a gene related to oxidative stress is not necessarily equally indicative of susceptibility to oxidative stress. In order to account for differences in the significance of various disorder-associated polymorphisms, a weighting factor can be assigned to each polymorphism detected in the methods and kits described herein. As indicated above, four genes (MnSOD, CZSOD, CAT, and GP) are known to have very significant roles in oxidative stress in humans. All else being equal, disorder-associated polymorphisms that occur in one of these four genes are more significant than polymorphisms that occur in genes having less significant roles in oxidative stress. Thus, a greater weighting factor can be assigned to these polymorphisms than to others. By way of example, the weighting factor assigned to these four polymorphisms can be 1 to 10 times greater than the weighting factor assigned to disorder-associated polymorphisms (having equal correlation with the corresponding disorder, as discussed below) in other genes. Preferably, the weighting factor assigned to polymorphisms in the MnSOD, CZSOD, CAT, and GP genes is twice that assigned to disorder-associated polymorphisms in other genes.

Another factor which can influence the significance that is assigned to occurrence of a disorder-associated polymorphism in a human's genome is the degree to which the polymorphism is correlated with the corresponding disorder. Some disorders are highly correlated with occurrence of a genetic polymorphism, and other disorders exhibit lower correlation with a polymorphism. When a polymorphism is reported to be associated with a disorder (i.e., with a disease or pathological condition), a degree of correlation between the polymorphism and the disorder is often reported. One useful way of calculating a factor that describes correlation between a polymorphism and a disorder is to calculate an odds ratio that describes the likelihood that an individual in whose genome the disorder-associate polymorphism occurs will exhibit or develop the disorder. Because the kits and methods described herein can be used to detect whether the human is homozygous for the disease-associated polymorphism, odds ratios calculated for homozygous individuals can also be used, if they are available. Odds ratios can be calculated as described in the art.

For a disorder-associated polymorphism, the odds ratio can be calculated as follows. First, the odds of being afflicted with the disorder are calculated for a first population in whom the polymorphism occurs by dividing the number of afflicted individuals in the first population by the total number of individuals in the first population. Second, the odds of being afflicted with the disorder are calculated for a first population in whom the polymorphism does not occur by dividing the number of afflicted individuals in the second population by the total number of individuals in the second population. Third, the odds ratio is calculated by dividing the odds for the first population by the odds for the second population. If the odds ratio is greater than one, then this is an indication that occurrence of the polymorphism is associated with occurrence of the disorder. Furthermore, the magnitude of the odds ratio is an indication of the significance of the association.

An overall oxidative stress susceptibility score for a human can be determined as follows. A significance score can be assigned to each disorder-associated polymorphism that is detected in the human's genome using a method or kit described herein. The significance score is a constant (e.g., 1.00), and is multiplied by any significance factor (e.g., 1-10, preferably 2, for the MnSOD, CZSOD, CAT, and GP genes) and by any correlation factor that is available. If information is available which describes the correlation between homozygosity for the polymorphism and the corresponding disorder, then that correlation factor should be used in place of the correlation factor for mere occurrence of the polymorphism, at least if the method or kit is used to rule out occurrence in the subject's genome of corresponding non-disorder-associated polymorphisms. If significance and correlation factors are not available, then values of 1.00 should be assigned to each. An overall score is determined by summing the significance score for each disorder-associated polymorphism that is detected using the method or kit. This overall oxidative stress susceptibility score can be compared with the values obtained from other subjects, or it can be compared with the value (i.e., zero) which would be expected to occur in a human whose genome does not include any disorder-associated polymorphism in a gene associated with oxidative stress.

By way of example, the Kimura reference describes two corresponding polymorphisms that occur in the MnSOD gene (i.e., occurrence of either C or T at a particular position in the MnSOD gene). Individuals in whose genome the disorder-associated polymorphism occur exhibit an odds ratio of 1.43 for the disorder (a form of macular degeneration), and individuals who are homozygous for the same polymorphism exhibit an odds ratio of 10.14. Thus, when the MnSOD gene is one of the genes assessed in the methods and kits described herein, a weighting factor of 1.43 can be applied to occurrence of this disorder-associated polymorphism in the subject's genome, and a weighting factor of 10.14 can be applied if the method or kit is used to determine that no other corresponding polymorphism occurs in the subject's genome. As indicated herein, an additional factor can be combined with this factor to represent the significance of the MnSOD gene in oxidative stress. Thus, if this latter factor is selected to be 2, then occurrence of the disorder-associated polymorphism described in Kimura can be assigned a significance of 2.86, and exclusive occurrence of that polymorphism (i.e., homozygosity) can be assigned a significance of 20.28.

The method used to assess occurrence of any particular disorder-associated polymorphism (or non-disorder-associated polymorphism) is not critical. Numerous methods of detecting occurrence of a polymorphism are known in the art, and substantially any of those methods can be used in the kits and methods described herein. Naturally, the reagents included in the kit will vary depending on the method to be used to detect the polymorphisms. Examples of some suitable polymorphism detection methods are provided below.

In one embodiment, a pair of oligonucleotide primers are used to amplify a portion of the gene that includes a polymorphic region. Detection of one or more of the polymorphisms that occur at the polymorphic region can be achieved by contacting the amplified portion with an oligonucleotide having a sequence that it will anneal under stringent conditions with the amplified portion only if one polymorphism occurs at the portion, but will not anneal with the amplified portion if another polymorphism occurs at that portion. Various acceptable stringent conditions are known in the art, and can be modified by the skilled artisan as appropriate to any particular amplified portion/oligonucleotide pair. An example of stringent conditions is hybridization in 6x sodium chloride/sodium citrate (SSC) at about 45°C, followed by one or more washes in 0.2x SSC, 0.1% (w/v) SDS at 50°C.

In an alternative embodiment, one or more molecular beacon oligonucleotides are used to detect polymorphisms (disorder-associated, non-disorder-associated, or both) in a sample that contains a copy of the subject's genome, a fraction of the subject's genome, or amplification products generated from the subject's genome (e.g., amplified portions of oxidative stress-associated genes in which portions polymorphisms are known to occur).

Molecular beacon probes are single-stranded oligonucleotides having a fluorescent label (e.g. rhodamine, FAM, TET, VIC, JOE, or HEX) attached to the 5'-end thereof and a fluorescence quencher (e.g. TAMRA or DABCYL) attached to the 3'-end thereof (or vice versa), as described (Kostrikis et al., 1998, Science 279:1228-1229). The sequence of each molecular beacon probe is selected to include two complementary hairpin regions, whereby the probe can self-anneal to form a hairpin structure. The 5'- and 3'- ends are brought into close association when the hairpin structure forms. The probe also comprises a targeting portion which is selected to be complementary to a target sequence (e.g. a single polymorphism of an oxidative-stress-associated gene). The targeting portion and at least one of the hairpin regions are located in close proximity to one another, meaning that the targeting portion either overlaps the hairpin region or flanks it, having no more than about 5 nucleotide residues therebetween.

If the hairpin regions of the molecular beacon probe anneal with one another, then the probe does not fluoresce, because the hairpin structure forms and the fluorescence quencher attached to one end of the probe quenches fluorescence of the label attached to the other end of the probe. If the targeting portion of the probe anneals with a region of a nucleic acid having the target sequence, then formation of the hairpin structure is inhibited, the fluorescence quencher is not brought into association with the fluorescent label, and the probe fluoresces. Multiple molecular beacon probes can be used in a single reaction mixture, and fluorescence associated with the probes can be differentiated if the molecular beacon probes are spectrally distinct.

Thus, in this embodiment, one or more molecular beacon probes are used, each having targeting portion which is complementary to a target region (e.g. 20 to 40 nucleotide residues, more preferably 20 to 30 residues) of one polymorphism of an oxidative stress-associated gene (e.g., one of the genes disclosed herein). If the polymorphism to be detected is a single nucleotide polymorphism (SNP), then the target region includes, and preferably is approximately centered around, the nucleotide residue at which the polymorphism occurs. More preferably, two such probes are used, one having a targeting region completely complementary to the target region of one polymorphism of the gene (e.g., one of two polymorphisms of an SNP), and the other having a targeting region completely complementary to the target region of a corresponding polymorphism of the gene (e.g., the other polymorphism of the SNP).

In yet another embodiment of how polymorphisms in an oxidative damage-associated gene can be assessed, oligonucleotide primers which are complementary to a region adjacent a characteristic residue of the polymorphism are extended using a polymerase enzyme, and the identity of the nucleotide residue that is added to the primer in the position complementary to the characteristic residue is determined. The primer can be extended in the presence of non-extendable nucleotide residues in order to ensure that a limited number of (or only one) nucleotide residues are incorporated into the primer. Methods of this type are known in the art (e.g., the SNP-IT® technology of Orchid Biocomputer, Inc.) and are described, for example in U.S. Patents numbers 6,013,431 and 6,004,744.

Methods of Assessing Susceptibility to Individual Disorders Associated with Oxidative Stress

An patient's susceptibility to oxidative stress is predictive of the patient's susceptibility to individual disorders that are associated with or known to be caused by oxidative stress and its physiological consequences. By way of example, development, progression, or both, of Alzheimer's disease is thought to be influenced by oxidative damage sustained by the patient's tissues, including brain tissues. The rate or likelihood of development and progression of oxidative damage-associated disorders can be estimated by assessing the overall susceptibility of a patient to oxidative stress.

Even though others may have associated susceptibility to oxidative stress-associated disorders with occurrence in a patient of a polymorphic form of a single gene (e.g., susceptibility of a patient to Alzheimer's disease has been associated with occurrence of a polymorphic form of the ApoE4 gene), it is believed that this disclosure is the first to correlate development or progression of such a disorder with occurrence in a patient of polymorphic forms of multiple genes, including genes for which there is no recognized link between a polymorphic form of the individual gene and the disorder. Thus, by way of example, even though others may have identified an Alzheimer's disease-associated polymorphism of a single gene (ApoE4), they did not associate susceptibility to Alzheimer's disease with occurrence of polymorphic forms of other genes. It is disclosed herein for the first time that susceptibility to Alzheimer's disease can be associated with occurrence in a patient of disorder-associated polymorphisms in genes that have no previously known connection with Alzheimer's disease. By way of example, susceptibility of a patient to Alzheimer's disease can be assessed by detecting occurrence in the patient of disorder-associated polymorphisms in any of the genes described herein, such as those in the groups herein designated a)-g).

The individual disorders for which susceptibility can be assessed using these methods are not limited to Alzheimer's disease. The methods can be used to assess susceptibility to substantially any disorder which is presently known to be associated with, caused by, or exacerbated by oxidative stress or the physiological consequences of oxidative stress. Examples of these disorders include arteriosclerosis; atherosclerosis; autoimmune disorders such as macular degeneration and psoriasis; bacterial, viral, fungal, and parasitic infections; age-related loss of skin thickness, resilience, and flexibility; allergies; hair loss; hair discoloration (e.g., graying); tumorigenesis; brain degenerative disorders such as Alzheimer's, Lou Gherig's, Huntington's, and Parkinson's diseases; other neurodegenerative disorders such as Friedreich's ataxia, hereditary spastic paraplegia, cerebellar degeneration, and amyotrophic lateral sclerosis; respiratory disorders such as asthma; inflammatory disorders such as pancreatitis.

Kits for Assessing Oxidative Stress

The invention includes a kit for assessing the relative susceptibility of a human to oxidative stress. The kit contains reagents for performing one or more of the methods described herein. The reagents used in certain embodiments of the methods described herein are indicated above. Reagents useful for performing those methods using a variety of alternative sample preparation and polymorphism detection methods or chemistries are apparent to the skilled artisan.

Kits for detecting polymorphisms in individual genes are known in the art, and the kit of the invention can have similar components. However, a critical feature of the kit is that it includes reagents that permit its user to detect disorder-associated polymorphisms in at least three genes associated with oxidative stress. Preferably the kit includes reagents that permit detection of disorder-associated polymorphisms in at least 4, 6, 8, 10, 15, 20, or 30 or more such genes.

In one embodiment, the kit includes a plurality of oligonucleotides which anneal under stringent conditions with a disorder-associated polymorphism of one of the genes, but not with a non-disorder associated-polymorphism. Each of the oligonucleotides is preferably attached to a surface in order to facilitate handling of the oligonucleotide. The oligonucleotides can be linked with a plurality of surfaces (e.g., oligonucleotides for a particular polymorphism being attached to a particle discrete from a particle to which oligonucleotides for another polymorphism are attached), or they can be attached to discrete regions of a single surface (e.g., as in the GENECHIP™ device of Affymetrix, Inc.). Annealing between individual oligonucleotides and the polymorphism corresponding thereto can be detected using standard methods. The kit can also comprise oligonucleotides that are useful as molecular beacon probes or as extendable primers.

In one embodiment, the kit further comprises a DNA collection kit or apparatus, such as that described in co-pending U.S. patent application number 09/302,623 (allowed). Advantageously, DNA collected using the kit or apparatus can be stored or archived, and subjected to additional testing as previously unknown polymorphisms are discovered in genes associated with oxidative stress, or as the significance of previously unappreciated polymorphisms is realized.

The invention also relates to a method of assessing the advisability that a human should employ a skin care product comprising an anti-oxidant ingredient. The method is performed as described herein for assessing the degree of oxidative stress in a human. If a higher level of oxidative stress is detected in the human (i.e., than a human not having a disorder-associated polymorphism in a gene identified herein), then it is advisable the human should employ a skin care product comprising an anti-oxidant ingredient. A greater degree of oxidative stress detected in a human (or a greater susceptibility score, as described herein) correlates with an increased advisability that the human should use such a skin care product, and also indicates that a greater dose of the anti-oxidant ingredient(s) should be included in the skin care product.

It will be appreciated by those skilled in the art that changes can made to the embodiments described above without departing from the broad inventive concept thereof.

This invention is not limited to the particular embodiments disclosed, and includes modifications within the spirit and scope of the present invention as defined by the appended claims.

## Claims

1. A method of assessing relative susceptibility of a human to oxidative damage, the method comprising assessing occurrence in the human's genome of disorder-associated polymorphisms in at least two genes selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress;
f) genes which encode a component of the human DNA repair system; and
g) genes which encode a protein associated with production of a toxic oxygen species by a macrophage or polymorphonuclear neutrophilic granulocyte and which are not genes of a), b), c), d), e), or f),
whereby occurrence of any of the polymorphisms is an indication that the human is more susceptible to oxidative damage than a human whose genome does not comprise the polymorphism, and whereby occurrence of a plurality of the polymorphisms is an indication that the human is even more susceptible to oxidative damage than a human whose genome does not comprise the polymorphisms.

2. The method of claim 1, wherein the genes are selected from the group consisting of a), b), c), and d).

3. The method of claim 1, wherein the genes are selected from the group consisting of a), b), and c).

4. The method of claim 1, wherein the genes include at least one gene which encodes a protein associated with production of a toxic oxygen species by a macrophage or polymorphonuclear neutrophilic granulocyte.

5. The method of claim 1, wherein the genes include a gene encoding a component of the phagocyte-specific NADPH-oxidase complex.

6. The method of claim 1, wherein the genes include a gene encoding a matrix metalloproteinase.

7. The method of claim 6, wherein the matrix metalloproteinase is selected from the group consisting of MMP-1, MMP-2, MMP-3, and MMP-7.

8. The method of claim 7, wherein the matrix metalloproteinase is MMP-1.

9. The method of claim 1, wherein the genes include a gene encoding a glutathione S-transferase.

10. The method of claim 9, wherein the glutathione S-transferase is selected from the group consisting of GSTP1, GSTT1, and GSTM1.

11. The method of claim 1, wherein the genes include the gene encoding tumor necrosis factor alpha.

12. The method of claim 1, wherein the genes include the gene encoding methylenetetrahydrofolate reductase.

13. The method of claim 1, wherein the genes include
i) the gene which encodes mitochondrial manganese superoxide dismutase (MnSOD),
ii) the gene which encodes cytoplasmic copper/zinc superoxide dismutase (CZSOD),
iii) the gene which encodes catalase, and
iv) the gene which encodes glutathione peroxidase.

14. The method of claim 1, wherein the genes are selected from the group consisting of
i) the gene which encodes MnSOD,
ii) the gene which encodes CZSOD,
iii) the gene which encodes catalase,
iv) the gene which encodes glutathione peroxidase,
v) the gene which encodes glutathione S-transferase P1 (GSTP1),
vi) the gene which encodes glutathione S-transferase theta 1 (GSTT1),
vii) the gene which encodes glutathione S-transferase M1 (GSTM1),
viii) the gene which encodes glutathione reductase,
ix) the gene which encodes thioredoxin reductase,
x) the gene which encodes paraoxonase,
xi) the gene which encodes NAD(P)H:quinone oxidoreductase 1,
xii) the gene which encodes 8-oxo-7,8-dihydrodeoxyguanosine triphosphatase,
xiii) the gene which encodes epoxide hydrolase,
xiv) the gene which encodes myeloperoxidase,
xv) the gene which encodes tumor necrosis factor alpha,
xvi) the gene which encodes NADH/NADPH oxidase p22 phox protein,
xvii) the gene which encodes nitric oxide synthase,
xviii) the gene which encodes xanthine oxidase,
xix) the gene which encodes cytochrome P450,
xx) the gene which encodes apolipoprotein E,
xxi) the gene which encodes UDP-glucuronosyltransferase 1A1,
xxii) the gene which encodes acid phosphatase,
xxiii) the gene which encodes protein phosphotyrosine phosphatase,
xxiv) the gene which encodes epinephrine oxidase,
xxv) the gene which encodes cystathionine beta-synthase,
xxvi) the gene which encodes cystathionine gamma-lyase,
xxvii) the gene which encodes N5-methyl THF:homocysteine methyltransferase,
xxviii) the gene which encodes methylenetetrahydrofolate reductase,
xxix) genes which encode an S-adenosylinethionine methyltransferase,
xxx) genes which encode a heat shock protein,
xxxi) genes encoding a component of the phagocyte-specific NADPH-oxidase complex, and
xxxii) genes which encode a matrix metalloproteinase.

15. The method of claim 14, wherein the genes are selected from the group consisting of i) through iv).

16. The method of claim 14, wherein the genes are selected from the group consisting of i) through xi).

17. The method of claim 14, wherein the genes are selected from the group consisting of i) through xvii).

18. The method of claim 14, comprising assessing occurrence in the human's genome of disorder-associated polymorphisms in at least four of i) through xxxii).

19. The method of claim14, comprising assessing occurrence in the human's genome of disorder-associated polymorphisms in at least six of i) through xxxii).

20. The method of claim14, comprising assessing occurrence in the human's genome of disorder-associated polymorphisms in at least ten of i) through xxxii).

21. The method of claim 14, comprising assessing occurrence in the human's genome of disorder-associated polymorphisms in at least fifteen of i) through xxxii).

22. The method of claim 1, wherein occurrence of an individual disorder-associated polymorphism is assessed by
contacting a nucleic acid derived from the human's genome with a first oligonucleotide that anneals with higher stringency with the disorder-associated polymorphism than with a corresponding non-disorder-associated polymorphism and
assessing annealing of the first oligonucleotide and the nucleic acid, whereby annealing of the first oligonucleotide and the nucleic acid is an indication that the human's genome comprises the disorder-associated polymorphism.

23. The method of claim 22, wherein the first oligonucleotide is attached to a support.

24. The method of claim 23, wherein the support has a plurality of different first oligonucleotides attached thereto.

25. The method of claim 23, wherein the support has attached thereto at least five first oligonucleotides that anneal with higher stringency with the disorder-associated polymorphisms than with the corresponding non-disorder-associated polymorphisms.

26. The method of claim 23, wherein the support has attached thereto at least ten first oligonucleotides that anneal with higher stringency with the disorder-associated polymorphisms than with the corresponding non-disorder-associated polymorphisms.

27. The method of claim 23, wherein the support has attached thereto at least fifteen first oligonucleotides that anneal with higher stringency with the disorder-associated polymorphisms than with the corresponding non-disorder-associated polymorphisms.

28. The method of claim 22, wherein the first oligonucleotide is a molecular beacon oligonucleotide.

29. The method of claim 22, wherein occurrence of an individual disorder-associated polymorphism is further assessed by
contacting the nucleic acid with a second oligonucleotide that anneals with higher stringency with a non-disorder-associated polymorphism than with the corresponding non-disorder-associated polymorphism and
assessing annealing of the second oligonucleotide and the nucleic acid, whereby annealing of the second oligonucleotide and the nucleic acid is an indication that the human's genome does not comprise the disorder-associated polymorphism.

30. The method of claim 29, wherein the second oligonucleotide is attached to a support.

31. The method of claim 30, wherein the first and second oligonucleotides are attached to the same support.

32. The method of claim 29, wherein the second oligonucleotide is a molecular beacon oligonucleotide.

33. The method of claim 32, wherein the first and second oligonucleotides are spectrally distinct molecular beacon oligonucleotides.

34. The method of claim 1, further comprising calculating a susceptibility score by summing, for each of the selected genes in which a disorder-associated polymorphism occurs in the human's genome, the product of a constant and a correlation factor, wherein the correlation factor represents the fraction of humans heterozygous or homozygous for the disorder-associated polymorphism who exhibit the corresponding disorder, whereby the susceptibility score represents the relative susceptibility of the human to oxidative damage.

35. The method of claim 34, wherein the same constant is used for each selected gene.

36. The method of claim 34, wherein the constant used for each gene of group a) is greater than the constant used for the genes of groups b), c), d), and e).

37. The method of claim 34, wherein the constant used for each gene of group a) is at least twice as great as the constant used for the genes of groups b), c), d), and e).

38. The method of claim 1, wherein each of the polymorphisms is a single nucleotide polymorphism (SNP).

39. The method of claim 1, wherein occurrence of a SNP is assessed by annealing a nucleic acid derived from the human's genome with a primer that is complementary to the region adjacent the SNP on its 3' side, extending the primer using a polymerase in order to add a nucleotide residue complementary to the SNP to the primer, and detecting the identity of the nucleotide residue complementary to the SNP.

40. The method of claim 39, wherein the nucleotide residue is a non-extendable residue.

41. A method of selecting a dose of an anti-oxidant composition for administration to a human, the method comprising assessing occurrence in the human's genome of disorder-associated polymorphisms in at least one gene selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress
f) genes which encode a component of the human DNA repair system; and
g) genes which encode a protein associated with production of a toxic oxygen species by a macrophage or polymorphonuclear neutrophilic granulocyte and which are not genes of a), b), c), d), e), or f),
whereby occurrence of any of the polymorphisms is an indication that a greater dose of the composition should be administered to the human; and
selecting a dose of the composition based on occurrence of the polymorphisms.

42. A kit for assessing relative susceptibility of a human to oxidative damage, the kit comprising reagents for assessing occurrence in the human's genome of disorder-associated polymorphisms in at least two genes selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress;
f) genes which encode a component of the human DNA repair system; and
g) genes which encode a protein associated with production of a toxic oxygen species by a macrophage or polymorphonuclear neutrophilic granulocyte and which are not genes of a), b), c), d), e), or f).

43. The kit of claim 42, wherein the reagents comprise first oligonucleotides that anneal with higher stringency with the disorder-associated polymorphisms than with corresponding non-disorder-associated polymorphisms.

44. The kit of claim 43, wherein each of the first oligonucleotides is attached to a support.

45. The kit of claim 44, wherein each of the first oligonucleotides is attached to the same support.

46. The kit of claim 44, wherein each of the first oligonucleotides is attached to a different support.

47. The kit of claim 43, wherein the first oligonucleotides are molecular beacon oligonucleotides.

48. The kit of claim 43, wherein the kit further comprises second oligonucleotides that anneal with higher stringency with the non-disorder-associated polymorphisms than with corresponding disorder-associated polymorphisms.

49. The kit of claim 48, wherein the first and second oligonucleotides are spectrally distinct molecular beacon oligonucleotide pairs.

50. The kit of claim 42, wherein the reagents comprise primers that are complementary to the region adjacent a characteristic residue of the disorder-associated polymorphism for amplifying at least the characteristic residue.

51. The kit of claim 50, further comprising a polymerase capable of extending the primers by adding a nucleotide residue complementary to the characteristic residue.

52. The kit of claim 51, further comprising a non-extendable nucleotide residue.

53. The kit of claim 42, further comprising an instructional material which includes a numerical value representing the product of a constant and a correlation factor, wherein the correlation factor represents the fraction of humans heterozygous or homozygous for the disorder-associated polymorphism who exhibit the corresponding disorder.

54. The kit of claim 53, wherein the same constant is used for each selected gene.

55. The kit of claim 53, wherein the constant used for each gene of group a) is greater than the constant used for the genes of groups b), c), d), and e).

56. The kit of claim 53, wherein the constant used for each gene of group a) is at least twice as great as the constant used for the genes of groups b), c), d), and e).

57. The kit of claim 42, wherein the genes are selected from the group consisting of
i) the gene which encodes MnSOD,
ii) the gene which encodes CZSOD,
iii) the gene which encodes catalase,
iv) the gene which encodes glutathione peroxidase,
v) the gene which encodes glutathione S-transferase P1 (GSTP1),
vi) the gene which encodes glutathione S-transferase theta 1 (GSTT1),
vii) the gene which encodes glutathione S-transferase M1 (GSTM1),
viii) the gene which encodes glutathione reductase,
ix) the gene which encodes thioredoxin reductase,
x) the gene which encodes paraoxonase,
xi) the gene which encodes NAD(P)H:quinone oxidoreductase 1,
xii) the gene which encodes 8-oxo-7,8-dihydrodeoxyguanosine triphosphatase,
xiii) the gene which encodes epoxide hydrolase,
xiv) the gene which encodes myeloperoxidase,
xv) the gene which encodes tumor necrosis factor alpha,
xvi) the gene which encodes NADH/NADPH oxidase p22 phox protein,
xvii) the gene which encodes nitric oxide synthase,
xviii) the gene which encodes xanthine oxidase,
xix) the gene which encodes cytochrome P450,
xx) the gene which encodes apolipoprotein E,
xxi) the gene which encodes UDP-glucuronosyltransferase 1A1,
xxii) the gene which encodes acid phosphatase,
xxiii) the gene which encodes protein phosphotyrosine phosphatase,
xxiv) the gene which encodes epinephrine oxidase,
xxv) the gene which encodes cystathionine beta-synthase,
xxvi) the gene which encodes cystathionine gamma-lyase,
xxvii) the gene which encodes N5-methyl THF:homocysteine methyltransferase,
xxviii) the gene which encodes methylenetetrahydrofolate reductase,
xxix) genes which encode an S-adenosylmethionine methyltransferase,
xxx) genes which encode a heat shock protein,
xxxi) genes encoding a component of the phagocyte-specific NADPH-oxidase complex, and
xxxii) genes which encode a matrix metalloproteinase.

58. A method of assessing the advisability that a human should employ a skin care product comprising an antioxidant, the method comprising assessing occurrence in the human's genome of disorder-associated polymorphisms in at least two genes selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress;
f) genes which encode a component of the human DNA repair system; and
g) genes which encode a protein associated with production of a toxic oxygen species by a macrophage or polymorphonuclear neutrophilic granulocyte and which are not genes of a), b), c), d), e), or f), whereby occurrence of any of the polymorphisms is an indication that it is more advisable for the human to employ a skin care product comprising an antioxidant than a human whose genome does not comprise the polymorphism, and whereby occurrence of a plurality of the polymorphisms is an indication that it is even more advisable that the human should employ a skin care product comprising an antioxidant than a human whose genome does not comprise the polymorphisms.

59. The method of claim 58, wherein the genes include a gene encoding a matrix metalloproteinase.

60. The method of claim 59, wherein the matrix metalloproteinase is selected from the group consisting of MMP-1, MMP-2, MMP-3, and MMP-7.

61. The method of claim 60, wherein the matrix metalloproteinase is MMP-1.

62. The method of claim 58, wherein the genes include a gene encoding a glutathione S-transferase.

63. The method of claim 62, wherein the glutathione S-transferase is selected from the group consisting of GSTP1, GSTT1, and GSTM1.

64. The method of claim 58, wherein the genes include the gene encoding tumor necrosis factor alpha.

65. The method of claim 58, wherein the genes include the gene encoding methylenetetrahydrofolate reductase.

66. A method of selecting a dose of an anti-oxidant composition for administration to a human in a skin care product, the method comprising assessing occurrence in the human's genome of disorder-associated polymorphisms in at least one gene selected from the group consisting of
a) genes which encode an enzyme that catalyzes conversion of a toxic oxygen species to a less toxic oxygen species;
b) genes which encode a protein that provides protection against oxidative stress;
c) genes which encode a protein that induces production of a toxic oxygen species;
d) genes which encode a protein that indirectly affects oxidative stress;
e) genes which encode a protein for which the level of expression of the protein is associated with oxidative stress;
f) genes which encode a component of the human DNA repair system; and
g) genes which encode a protein associated with production of a toxic oxygen species by a macrophage or polymorphonuclear neutrophilic granulocyte and which are not genes of a), b), c), d), e), or f),
whereby occurrence of any of the polymorphisms is an indication that a greater dose of the composition should be administered to the human in the skin care product; and
selecting a dose of the composition for the skin care product based on occurrence of the polymorphisms.

67. The method of claim 66, wherein the genes include a gene encoding a matrix metalloproteinase.

68. The method of claim 67, wherein the matrix metalloproteinase is selected from the group consisting of MMP-1, MMP-2, MMP-3, and MMP-7.

69. The method of claim 68, wherein the matrix metalloproteinase is MMP-1.

70. The method of claim 66, wherein the genes include a gene encoding a glutathione S-transferase.

71. The method of claim 70, wherein the glutathione S-transferase is selected from the group consisting of GSTP1, GSTT1, and GSTM1.

72. The method of claim 66, wherein the genes include the gene encoding tumor necrosis factor alpha.

73. The method of claim 66, wherein the genes include the gene encoding methylenetetrahydrofolate reductase.
